# EUROPEAN PATENT APPLICATION

(11) **EP 2 165 679 A1**
(43) Date of publication of application: **24.03.2010**
(21) Application number: 08765264.0
(22) Date of filing: 06.06.2008
(51) Int. Cl.: A61F 2/16, A61F 9/007

(54) **INTRAOCULAR LENS INSERTING TOOL, AND INTRAOCULAR LENS HOUSING TYPE INSERTING TOOL**

(30) Priority: 06.06.2007 JP 2007150681
(71) Applicant: STAAR Japan Inc., Urayasu-shi Chiba 279-0012 (JP)
(72) Inventor: KOBAYASHI, Kenichi, Ichikawa-shi Chiba 272-0001 (JP); TOYOMANE, Shinobu, Ichikawa-shi Chiba 272-0001 (JP); YOSHIDA, Katsumi, Ichikawa-shi Chiba 272-0001 (JP)
(74) Representative: TBK-Patent
(86) International application number: PCT/JP2008/060451
(87) International publication number: WO 2008/149978

(57) **Abstract**

The insertion device (2) includes a main body (3) including a lens housing portion (3b) in which an intraocular lens (1) is housed and an insertion cylindrical portion (3a) for ejecting the lens into the eye through its front end opening (3j), a pushing shaft (4) configured to move the lens from the lens housing portion to push out the lens into the eye through the insertion cylindrical portion, and a liquid flow path (10) configured to cause liquid filled in an inside of the main body to flow from the inside of the main body to an outside thereof, the liquid flow path not including the front end opening (3j). The pushing shaft includes a structure configured to shut off the liquid flow path after the lens is ejected from the front end opening and thereby flow of the liquid through the front end opening is allowed.

## Description

### TECHNICAL FIELD

The present invention relates to an insertion device for inserting an intraocular lens into an eye, the intraocular lens being inserted thereinto instead of a crystalline lens after the crystalline lens is extracted because of cataract or in order to cure abnormal refraction.

### BACKGROUND ART

In current cataract surgeries, first, a central portion of an anterior capsule of an eye (eyeball) is ablated. Next, a clouded crystalline lens is crushed (emulsified) and removed. Then, an artificial intraocular lens (hereinafter simply referred to as "lens") is placed at a position of the removed clouded crystalline lens.
When the lens is placed in the eyeball, the lens is deformed to be small by folding it or the like by utilizing flexibility of the lens and inserted into the eyeball through a small incision formed thereon.

In an actual surgery, a dedicated insertion device is frequently used which deforms the lens set in a main body of the device into a small shape while moving the lens in the main body by a pushing shaft, and pushes out the lens into the eyeball from a front end opening of an insertion cylinder (nozzle) inserted into the incision. Such an insertion device is used not only for the cataract surgery but also for a lens inserting surgery for an eyesight correction medical treatment.

When the lens is inserted into the eyeball by using the insertion device, a viscoelastic material such as sodium hyaluronate is introduced into the main body of the insertion device as a lubricant such that the lens is smoothly moved and deformed in the insertion device (see Japanese Patent Laid-Open No. 2004-351196). Moreover, the viscoelastic material introduced into the eyeball through the insertion cylinder has a function of swelling (spreading) a space in an anterior chamber of the eyeball into which the lens will be inserted. In addition, it has been recently required to use inexpensive physiologic saline in place of the viscoelastic material.

However, when moving the lens by the pushing shaft while folding it small in the insertion cylinder, most of or entire space inside the insertion cylinder is occupied by the lens, which does not allow liquid (viscoelastic material or physiologic saline) in the main body to flow out to the outside. In this case, pressure of the liquid in the main body increases, which may bring an impossibility of performing pushing of the pushing shaft, that is, pushing-out of the lens into the eye smoothly.

Therefore, it is preferable that not only the front end opening of the insertion cylinder, but also a flow path through which the liquid in the main body is flowed out to the outside be formed in the insertion device. However, when the lens is injected from the front end opening of the insertion cylinder in a state in which the anterior chamber is swelled with the physiologic saline whose viscoelasticity is low and which has been discharged from the insertion cylinder, pressure in the main body is lower than that in the eyeball due to existence of the flow path, which brings about a risk that aqueous fluid in the eyeball may flow back to the inside of the main body.

### DISCLOSURE OF INVENTION

The present invention provides an insertion device capable of smoothly performing pushing-out of the lens into the eye by the pushing shaft in the state in which the main body is filled with the liquid, and of preventing the aqueous fluid in the eyeball from flowing back to the inside of the insertion device.

The present invention provides as one aspect thereof an insertion device for inserting an intraocular lens into an eye which includes a main body configured to include a lens housing portion in which the lens is housed and an insertion cylindrical portion for ejecting the lens into the eye through its front end opening, a pushing shaft configured to move the lens from the lens housing portion to push out the lens into the eye through the insertion cylindrical portion, and a liquid flow path other than the front end opening that is to cause liquid to flow from the inside of the main body to the outside or inside the main body. The insertion device is configured to open the liquid flow path when the lens moves toward the front end opening in the insertion cylindrical portion, and to shut off the liquid flow path after the lens is ejected from the front end opening and inserted into the eye.

The present invention provides as another aspect thereof an intraocular lens preloaded insertion device including the above-described insertion device, and an intraocular lens held in a lens housing portion of the insertion device.

The present invention provides as still another aspect thereof a method for manufacturing an intraocular lens preloaded insertion device comprising the steps of preparing the above-described insertion device, and causing a lens housing portion of the insertion device to hold the intraocular lens.

Other aspects of the present invention will become apparent from the following description and the attached drawings.

### BRIEF DESCRIPTION OF THE DRAWINGS

FIG. 1 shows a top view and a side view of an insertion device which is a first embodiment (Embodiment 1) of the present invention.

FIG. 2 shows a top view and a side view showing the insertion device of Embodiment 1 before assembly.

FIG. 3 shows a top view of the insertion device of Embodiment 1 in a state in which a lens is removed therefrom.

FIG. 4 shows a side view of a front end position of a support portion for supporting the lens in the insertion device of Embodiment 1.

FIG. 5 shows a sectional view of a lens housing portion in the insertion device of Embodiment 1.

FIG. 6 is a top view showing a state in which the lens is moving in a nozzle portion of the insertion device of Embodiment 1.

FIG. 7 is a top view showing a state in which the lens is injected from the nozzle portion of the insertion device of Embodiment 1.

FIG. 8 is a top view showing a state in which a lens is injected from a nozzle of a conventional insertion device.

FIG. 9 shows a state in which the nozzle portion is inserted into an eye in the insertion device of Embodiment 1.

FIG. 10 shows a sectional view of the nozzle portion and the lens housing portion of the insertion device of Embodiment 1.

FIG. 11 shows a top view and a sectional view of the nozzle portion inside which the lens is moved and a space is formed.

FIG. 12 shows a top view and a sectional view of the nozzle portion inside which the lens is moved and no space is formed.

FIG. 13 shows a situation in which the insertion device of Embodiment 1 is stored.

FIG. 14 shows a top view of an insertion device that is a second embodiment (Embodiment 2) of the present invention.

FIG. 15 shows a sectional view of a main body of the insertion device of Embodiment 2.

FIG. 16A shows a sectional view of the insertion device of Embodiment 2 in an initial state.

FIG. 16B is a sectional view showing a state in which a lens is moving in a nozzle portion in the insertion device of Embodiment 2.

FIG. 17 is a sectional view of the main body and a seal cap of the insertion device in the state of FIG. 16B;

FIG. 18 is a sectional view showing a state in which the lens is injected from the nozzle portion in the insertion device of the second embodiment;

FIG. 19 is a sectional view of an insertion device which is a modified example of Embodiment 2 in an initial state.

FIG. 20 is a sectional view of a main body and a seal cap of the insertion device of the modified example.

FIG. 21 shows a flowchart of a method for manufacturing an intraocular lens preloaded insertion device of each of Embodiments 1 and 2.

### DESCRIPTION OF EMBODIMENT

Exemplary embodiments of the present invention will hereinafter be described with reference to the accompanying drawings.

### [Embodiment 1]

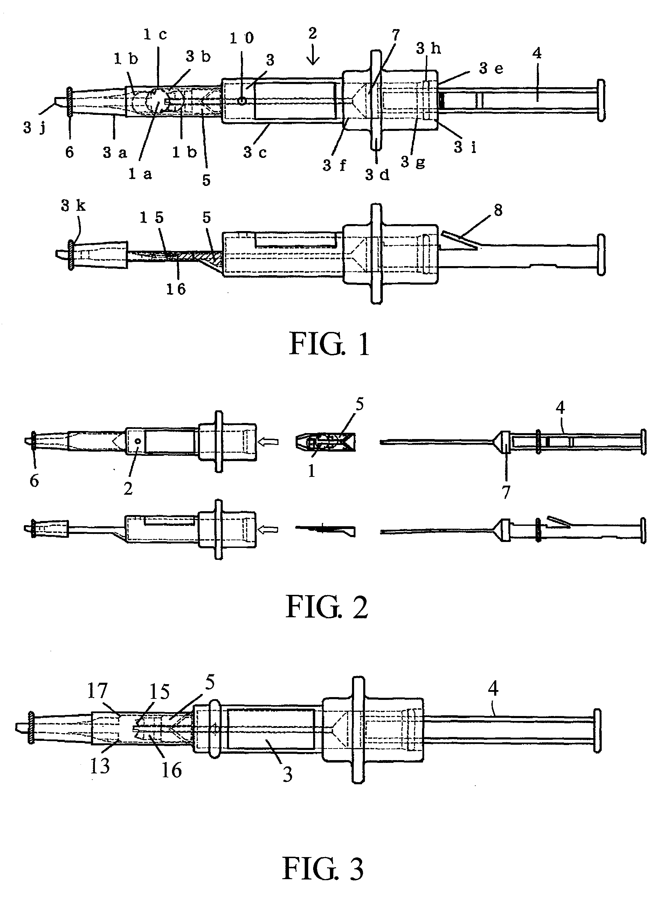

FIG. 1 shows an insertion device 2 for an intraocular lens (hereinafter simply referred to as "lens") which is a first embodiment (Embodiment 1) of the present invention. An upper part in FIG. 1 shows a top view, and a lower part shows a side view. FIG. 2 shows the insertion device before assembly. An upper part in FIG. 2 shows a top view and a lower part shows a side view.

In the description below, a nozzle side is referred to as "front (end) side", and a side opposite to the nozzle side is referred to as "rear (end) side". A direction extending toward the front and rear sides is referred to as "axial direction" of the insertion device, and a direction perpendicular to the axial direction is referred to as "up-and-down direction", "right-and-left direction" or "radial direction". Further, an axis extending parallel to the axial direction and passing through an inner space of the main body or a center of the lens is referred to as "central axis", and a direction around the central axis is referred to as "circumferential direction".

The insertion device 2 is basically constituted by a pushing shaft 4 and a main body 3 with a nozzle (hereinafter simply referred to as "main body").

The pushing shaft 4 has a small outer diameter so as to be able to pass through an inside of a nozzle portion 3a, and has at its front end a lens grip portion 4a vertically bifurcated. The lens grip portion 4a vertically holds a rear end of an optical portion 1a of a lens 1 in a lens housing portion 3b. This allows the lens 1 to be reliably pushed by the pushing shaft 4 in the front end direction.

The lens 1 has a circular shape in top view, and includes an optical portion 1a having a function of a lens and two support portions 1b extending from both sides (right and left sides) of the optical portion 1a. Each of the right and left support portions 1b is a wire-like portion that elastically supports the optical portion 1a in an eye (eyeball) after the lens 1 is inserted into the eyeball. A ring-shaped marginal portion 1c having upper and lower surfaces parallel to each other is formed around (that is, at a periphery of) the optical portion 1a. The marginal portion 1c is hereinafter referred to as "lens marginal portion 1c".

A seal cap 7 made of rubber is attached to an outer circumference of the pushing shaft 4. The seal cap 7 slides along an inner surface of the main body 3 with movement of the pushing shaft 4. The seal cap 7 is configured to provide appropriate sliding feeling (operational resistance feeling) to an operation of the pushing shaft 4, and to prevent liquid housed in the main body 3 from leaking out to the rear side.

The main body 3 includes an outer cylindrical portion 3c as a hand-held portion having an outer diameter suitable for holding the insertion device 2 by hand, a lens housing portion 3b provided closer to a front end of the main body 3 than the outer cylindrical portion 3c and housing a lens holding member 5, and a nozzle portion 3a as an insertion cylindrical portion provided closer to the front end than the lens housing portion 3b.

In a rear part of the outer cylindrical portion 3c, a flange portion 3d is formed as a portion supported by hand when pushing the pushing shaft 4.

The main body 3 has a hollow shape, and the lens 1, the lens holding member 5 and the pushing shaft 4 are inserted into the main body 3 through a rear end opening 3e thereof.

The outer cylindrical portion 3c includes, from its front end to a position closer to its rear end than the flange portion 3d, a first inner circumferential surface 3f having a cylindrical shape. At a part closer to the rear end than the first inner circumferential surface 3f, a second inner circumferential surface 3g having a cylindrical shape and a slightly smaller inner diameter than that of the first inner circumferential surface 3f is formed. Further, at a part closer to the rear end than the second inner circumferential surface 3g, a conical surface 3h is formed which has an increasing inner diameter toward the rear end. At a part from a rear end of the conical surface 3h to the rear end opening 3e to the rear end opening 3e, a third inner circumferential surface 3i having a cylindrical shape and a larger inner diameter than that of the first inner circumferential surface 3f is formed.

The nozzle portion 3a has decreasing inner and outer diameters toward the front end, and its part having a predetermined length from a front end opening 3j of the nozzle portion 3a toward the rear end is formed to be a thinnest part thereof which is an inserting part to be inserted into the eyeball through an incision formed on the eyeball. On an outer circumference of a rear end of the inserting part, a cover ring (O-ring) 6 made of an elastic member such as rubber is mounted. On a rear side of the cover ring 13 in the nozzle portion 3a, a step 3k is formed having a larger outer diameter than that of the inserting part for preventing rearward movement of the cover ring 6.

The lens housing portion 3b basically has a hollow flat plate shape having a vertical dimension smaller than a lateral dimension when viewed from the axial direction. A rear part of a lower surface of the lens housing portion 3b near a boundary between the lens housing portion 3b and the outer cylindrical portion 3c has a semi-conical shape having an increasing diameter toward the rear for reinforcement. Since the lens holding member 5 is inserted into the main body 3 through the rear end opening 3e, a continuous tapered connection part between an inner surface of the outer cylindrical portion 3c and an inner surface of the lens housing portion 3b provides an insertion guiding shape, thereby facilitating insertion of the lens holding member 5 into the main body 3.

The lens housing portion 3b can receive the insertion of the lens holding member 5 from the rear end thereof, and has an inner surface shape capable of stably holding the inserted lens holding member 5.

The insertion device 2 of this embodiment is an intraocular lens preloaded insertion device which is shipped in a state in which the lens 1 is installed (loaded) in the lens housing portion 3b from a factory, and is to be stored in a hospital until a surgery.

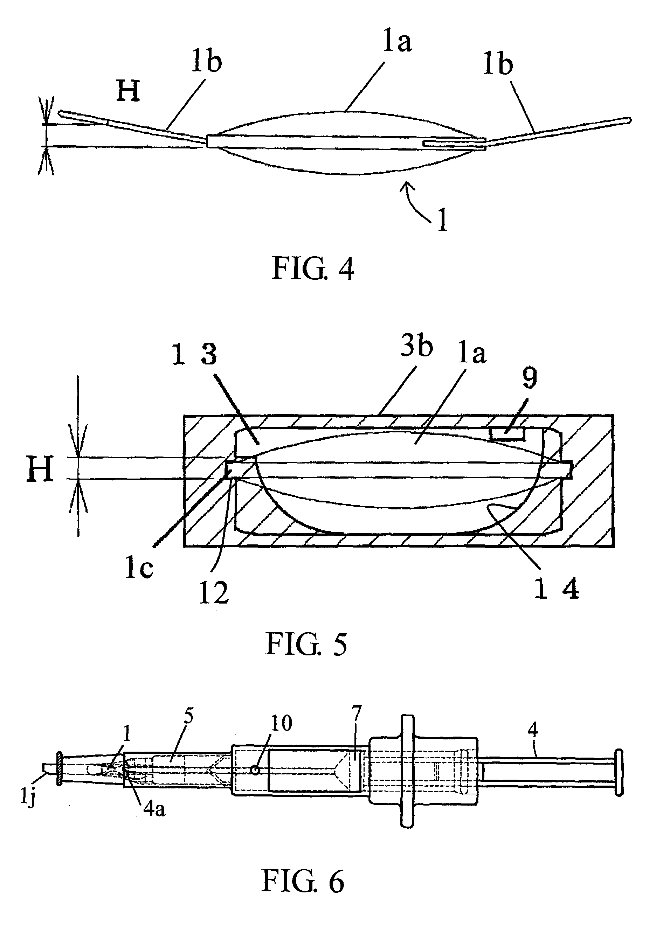

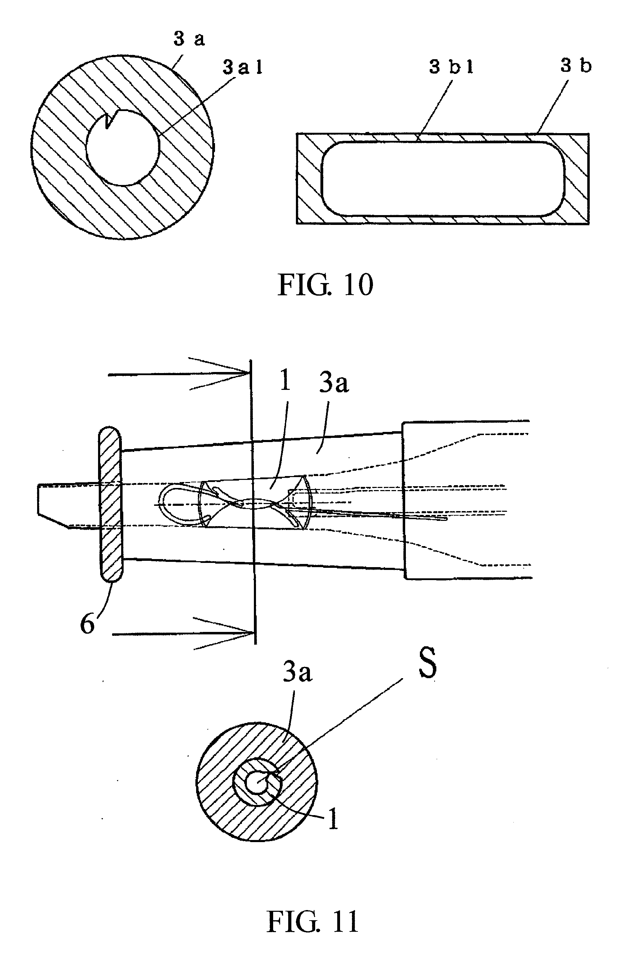

FIG. 5 shows a section orthogonal to the axial direction of the lens housing portion 3b. Further, FIG. 10 shows sections orthogonal to the axial direction of the nozzle portion 3a and the lens housing portion 3b. As shown in FIG. 10,circumferential walls 3b1 and 3a1 are formed from the lens housing portion 3b to the nozzle portion 3a so as to be an integral wall without an opening and a gap. In other words, four side (upper, lower, right and left) walls surrounding a space thereinside are circumferentially connected, and integrally formed without a hole, a gap and the like. Further, even in a case where sealing processing is applied to a gap generated out of need, it is regarded as that the lens housing portion 3b has a configuration without an opening and a gap, which is the same as that shown in FIG. 5 and FIG. 10.

This embodiment describes the case where the main body 3 is an integrally formed member such that at least the circumferential walls 3b1 and 3a1 formed from the lens housing portion 3b to the nozzle portion 3a have no opening or gap. However, an alternative embodiment of the present invention is not limited to this case. For example, the main body 3 may be constituted by joining by thermal welding or bonding two divided upper and lower members from their front end to their rear end to be integrated such that the main body 3 after its completion (before insertion of the lens holding member 5 into the main body 3) is an integral member without an opening and a gap at least in the circumferential walls from the lens housing portion 3b to the nozzle portion 3a.

Further, the main body 3 may be constituted by joining by thermal welding or bonding the lens housing portion 3b, nozzle portion 3a and outer cylindrical portion 3c produced separately from each other to be integrated such that the main body 3 after its completion (before insertion of the lens holding member 5 into the main body 3) is an integral member without an opening and a gap at least in the circumferential walls from the lens housing portion 3b to the nozzle portion 3a.

As shown in the top view of FIG. 1, a small hole 10 is formed on the circumferential wall of the outer tube portion 3c near its frond end. Before use of the insertion device 2, liquid such as low-viscoelastic physiologic saline can be introduced (filled) into the inside of the main body 3 through the hole 10.

In a case where there is a space S extending in the axial direction in the nozzle portion 3a as shown in FIG. 11 when the lens 1 is moved toward the front end opening 3j in the nozzle portion 3a, it is necessary to cover the hole 10 because it is necessary to cause the liquid existing in the main body 3 to flow out from the front end opening 3j of the nozzle portion 3a with the pushing of the pushing shaft 4.

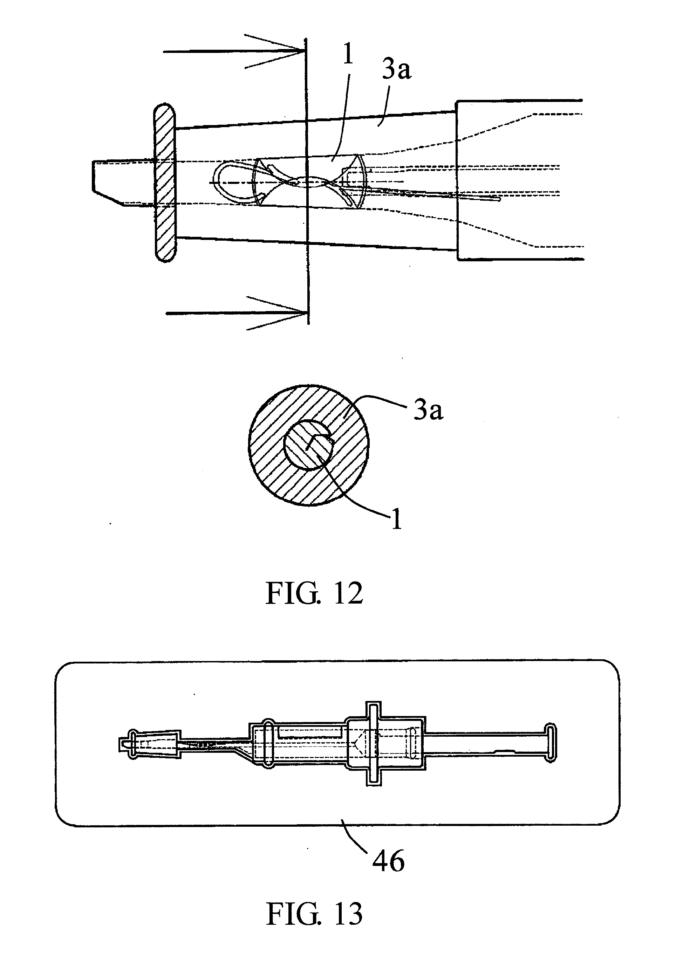

On the other hand, as shown in FIG. 12, in a case where there is almost no space or not a space extending in the axial direction because most of or entire inner space in the nozzle portion 3a is occupied by the lens 1 when the lens 1 is moved toward the front end opening 3j in the nozzle portion 3a, the liquid is confined in an area closer to the front end (front end opening 3j) than the seal cap 7 in the inside of the main body 3 (hereinafter, the area is referred to as "first area" in the main body 3, the area shown in FIG. 12 being an area between the seal cap 7 and the lens 1) by the pushing of the pushing shaft 4. This makes it impossible to push the pushing shaft 4 and push out the lens 1 smoothly. In this case, in order to allow the liquid in the first area in the main body 3 to flow out through the hole 10 to the outside of the main body 3, it is necessary to open the hole 10.

Because a width of the incision formed on the eyeball in a surgery is preferably small, it is necessary to make an outer diameter of the nozzle portion 3a small in accordance with the width of the incision. Therefore, a state as shown in FIG. 12 is often brought about.
Accordingly, forming (opening) a liquid flow path allowing the liquid to flow to the outside of the main body 3 through the hole 10 from the first area in the main body 3(that is, from the inside of the main body 3) enables smooth pushing of the pushing shaft 4 and smooth pushing-out of the lens 1. The liquid flow path herein does not include the front end opening 3j.

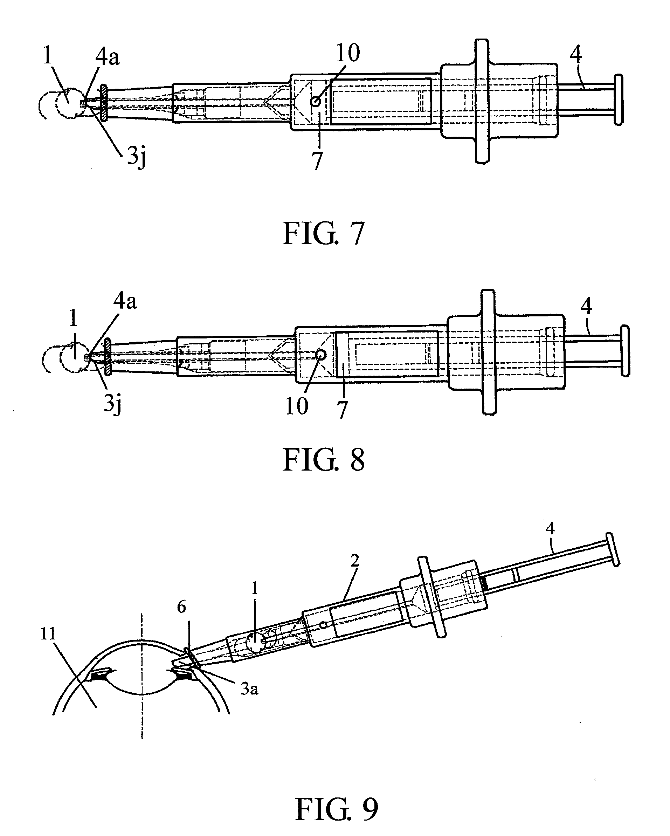

Further, in this case, the low-viscoelastic liquid existing in the first area in the main body 3 flows out also through the front end opening 3j of the nozzle portion 3a with the pushing of the pushing shaft 4. This causes, as shown in FIG.9, the liquid to flow into an eyeball 11 through the nozzle portion 3a inserted into an incision formed in the eyeball 11 to swell a space in an anterior chamber of the eyeball, which is effective for easy insertion of the lens 1 into the eyeball.

Next, a procedure for inserting the lens 1 into the eyeball will be described. As shown in FIG. 9, the pushing of the pushing shaft 4 is started from a state in which the nozzle portion 3a is inserted into the eyeball through the incision formed thereon. When the lens 1 is still located in the lens housing portion 3b, there is a space in the nozzle portion 3a. Therefore, in a state where the hole 10 is covered with a finger, the liquid in the first area in the main body 3 can be introduced into the eyeball to swell the space in the anterior chamber.

Thereafter, in a state where the lens 1 enters into the nozzle portion 3a and there is almost no space or not a space in the nozzle portion 3a as shown in FIG. 6, the finger covering the hole 10 is released from the hole 10 to open the hole 10. Thereby, it is possible to perform pushing of the pushing shaft 4 and pushing-out of the lens 1 smoothly as described above. In this way, the lens 1 is moved in the nozzle portion 3a to be injected (inserted) into the eyeball through the front end opening 3j.

However, as shown in FIG. 8, if the seal cap 7 attached to the pushing shaft 4 is located closer to the rear end than the hole 10 when the lens 1 is injected through the front end opening 3j, because the hole 10 is open, pressure in the first area in the main body 3 (in an area closer to the front end opening 3j than the seal cap 7 in the main body 3) may be reduced to be lower than pressure (eye pressure) in the eyeball 11. This may cause aqueous fluid in the eyeball to flow back into the first area in the main body 3.

In other words, since the inside of the eyeball is connected with the liquid flow path opened so as to allow the liquid to flow from the first area in the main body 3 (inside of the main body 3) to the outside thereof through the hole 10 via the front end opening 3j, the aqueous fluid in the eyeball flows into the first area in the main body 3 in which the pressure is low from the inside of the eyeball in which the pressure is high.

If such flow-back of the aqueous fluid is allowed, the pressure in the eyeball is greatly decreased, and thereby the space in the anterior chamber is rapidly changed from a swelled (expanded) state to a shrunken (narrowed) state. Further, with the back-flow of the aqueous fluid, movement (movement from a mydriatic state to a myotic state) of an iris of the eyeball is caused. Thereby, raising the possibility that the nozzle portion 3a or the lens grip portion 4a of the pushing shaft 4 contacts corneal endothelium or posterior capsule of the eyeball, which is unfavorable.

Then, is this embodiment, as shown in FIG. 9, in a state in which the pushing shaft 4 is not yet pushed in or the pushing shaft 4 is already pushed in but the lens 1 is still located in the lens housing portion 3b, the seal cap 7 attached to the pushing shaft 4 is set to be located closer to the rear end than the hole 10. Further, as shown in FIGS. 6 and 11, when the lens 1 is moved toward the front end opening 3j in the nozzle portion 3a (when there is almost no space or not a space in the nozzle portion 3a), the seal cap 7 is also set to be located closer to the rear end than the hole 10 to open the liquid flow path.

However, as shown in FIG. 7, when the lens 1 has passed through the inside of the nozzle portion 3a to be injected (inserted) into the eye through the front end opening 3j, the hole 10 is covered with the seal cap 7 or the seal cap 7 is caused to be located closer to the front end than the hole 10. This structure makes it possible to shut off the liquid flow path formed in the first area in the main body 3 including the hole 10 as an exit opening, which blocks the flow of the liquid from the front end opening 3j to the hole 10 (that is, to the outside).

In other words, in this embodiment, the insertion device 2 is configured to be capable of controlling opening and shut-off of the liquid flow path with the seal cap 7 as a sealing member or a shut-off member in accordance with a push-in position of the pushing shaft 4.

In the state shown in FIG. 7, the hole 10 is covered with the seal cap 7 to shut off the liquid flow path, which causes the first area in the main body 3 to be opened through only the front end opening 3j. In this state, because the inside of the first area in the main body 3 is filled with the liquid, even in a case where the pressure in the eyeball is higher than the pressure in the first area in the main body 3, the aqueous liquid in the eyeball does not flow back to the inside of the main body 3. Thereby, the pressure in the eyeball is maintained, and therefore the space in the anterior chamber is not greatly reduced, which hardly causes the movement in the iris.

Next, holding of the lens 1 in the lens housing portion 3b in the insertion device of this embodiment will be described. In the embodiment, as shown in FIG. 3 except for the lens 1, prevention of forward positional displacement of the lens 1 before pushing the pushing shaft 4 (in an initial state) and keeping of an angle of the front side support portion 1b are performed with an inner wall (17, 13) of the main body 3 (lens housing portion 3b), and prevention of positional displacement of the lens 1 rearward and keeping of an angle of the rear side support portion 1b are performed with the lens housing member 5.

As shown in FIG. 5, a lens holding groove portion 12 is formed at an intermediate part in the up-and-down direction in the inner wall of the lens housing portion 3b. The lens marginal portion 1c of the lens 1 (optical portion 1a) is engaged with the lens holding groove portion 12 to hold the lens 1, which positions the lens 1 in a height direction in the lens housing portion 3b.

Further, a front end of the front side support portion 1b is hooked on a support portion fixing groove portion 13 shown in FIGS. 5 and 3, which supports the front side support portion 1b so as to keep a natural angle (angle to the optical portion 1a in a state where no stress is applied to the lens 1). In detail, a height of the support portion fixing groove portion 13 with respect to the lens holding groove portion 12 is set to "H" such that the front end of the front side support portion 1b is located higher by a height "H" than the optical portion 1a to keep the natural angle. The rear side support portion 1b is supported so as to keep the natural angle in the same way by an inclined surface 16 (refer to FIG. 3) formed on the lens holding member 5.

In this way, in the initial state, only the lens marginal portion 1c is supported in the optical portion 1a and the front and rear side support portions 1b are supported so as to maintain their natural angles. This enables holding of the lens 1 in a state where a stress by its own weight or an external force is not substantially applied to the lens 1. The term "the state in which a stress is substantially not applied to the lens 1" denotes not only a state in which no stress is applied to the lens 1 at all, but also a state in which a minute stress is applied thereto such that a deformation influencing an optical function of the lens 1 (optical portion 1a) after insertion of the lens 1 into the eye does not occur even if the lens 1 is stored for a long time. In other words, the state denotes a state in which a stress or a deformation influencing the optical function of the lens 1 does not occur.

The lens holding groove portion 12 and the support portion fixing grove portion 13 respectively become smaller toward the front in the axial direction, and disappear near a rear end of the nozzle portion 3a. This allows the optical portion 1a to be deformed into a downward convex shape along a concave bottom surface 14 of the lens housing portion 3b when the lens 1 is moved toward the nozzle portion 3a, and allows a deformation of the front side support portion 1b with the deformation of the optical portion 1a. In this way, deforming the lens 1 to some extent at a position closer to the rear end than the nozzle portion 3a makes it possible to smoothly fold the lens 1 small in the nozzle portion 3a. A protrusion 9 formed on an upper inner surface of the lens housing portion 3b has a function of guiding the lens 1 held in the lens housing portion 3b to be a downward convex shape.

Further, prevention of forward positional displacement of the lens 1 in the axial direction is performed by that a vertical surface 17 is formed on the inner wall of the lens housing portion 3b to cause the lens marginal portion 1c to contact the vertical surface 17.

On the other hand, as shown in FIG. 3, the lens holding member 5 has a bifurcated portion 15 split into upper and down sides so as to sandwich the optical portion 1a loosely on its front end. This prevents rearward positional displacement of the lens 1 in the axial direction in the initial state.

The lens holding member 5 is moved together with the lens 1 toward the nozzle portion 3a in the lens housing portion 3b in association with the pushing of the pushing shaft 4 from the initial state. Then, contact of a front end of the lens holding member 5 to the vertical surface 17 of the lens housing portion 3b blocks a further movement of the lens holding member 5 to a nozzle portion side. Thereafter, the lens 1 pushed by the pushing shaft 4 singularly is moved and folded small in the nozzle portion 3a.

In order to maintain the initial state, it is necessary that the pushing shaft 4 is not pushed into the main body 3. Therefore, in this embodiment, as shown in FIG. 1, a lock portion 8 which can contact the main body 3 is provided on the pushing shaft 4 or resistance of the seal cap 7 provided on the pushing shaft 4 against the main body 3 is increased. Further, as shown in FIG. 13, the insertion device 2 in the initial state is contained in a case 46 from a factory shipment of the insertion device 2 up to intermediately before a surgery (which is also called as "in transportation"), and an inner surface shape of the case is made to correspond to a position and a shape of the pushing shaft 4 in the insertion device 2 in the initial state, the pushing shaft 4 can be fixed to the main body 12.

### [Second Embodiment]

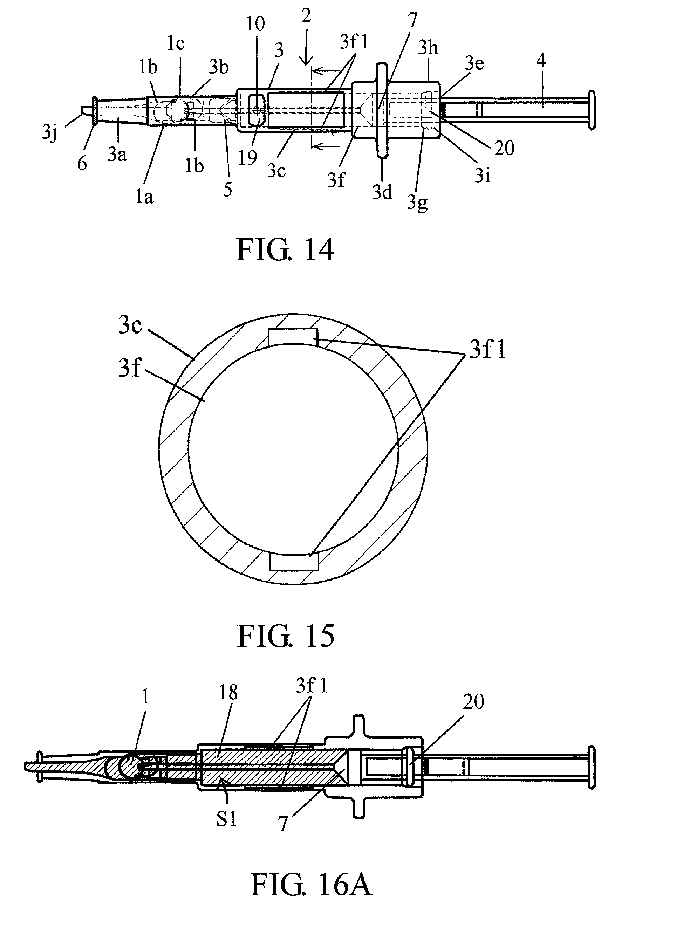

In the insertion device of Embodiment 1, the case has been described where the liquid filled in the first area in the main body is caused to flow to the outside of the main body 3 through the hole 10. In contrast thereto, in an insertion device shown in FIG. 14 which is a second embodiment (Embodiment 2) of the present invention, the liquid filled in the first area in the main body is caused to flow inside the main body 3.

FIG. 14 shows a top view of the insertion device 2 of this embodiment. In this embodiment, components having the same or similar functions as those of Embodiment 1 are denoted by the same reference numerals as those in Embodiment.

In this embodiment as well, the hole 10 is formed in the main body 3. However, after liquid such as physiologic saline is introduced into the main body 3 (first area in the main body 3) through the hole 10, the hole 10 is covered with the seal 19 or a finger.

As shown in a sectional view orthogonal to the axial direction (position of the section is shown by a dashed line in FIG. 14) of FIG. 15 and an axial sectional view of FIG. 16A, groove portions 3f1 extending in the axial direction are formed in the first inner circumferential surface 3f of the main body 3. Further, the rear end opening 3e of the main body 3 is closed by press contact of a seal ring 20 to the conical surface 3h formed on the main body 3, the seal ring 20 being attached on the pushing shaft 4 so as to be movable in the axial direction.

FIG. 16A shows the initial state described in Embodiment 1. Reference character S1 denotes an area in the main body 3 which is a first area (front end opening side area) closer to the front end than the seal cap 7 in the main body 3. Reference numeral 18 denotes liquid such as physiologic saline. In the initial state, there is the liquid 18 only in the first area S1 in the main body 3 partitioned by the seal cap 7 attached on the pushing shaft 4. Further, in this initial state, the entire groove portions 3f1 face only the first area S1.

When the lens 1 is moved toward the nozzle portion 3a in the main body 3 by pushing the pushing shaft 4 from the initial state (that is, during the movement of the lens 1 in the nozzle portion 3a), the seal cap 7 is located at the rear end of the groove portions 3f1. Thereby, the liquid in the first area S1 can be introduced into an eye to swell a space of an anterior chamber.

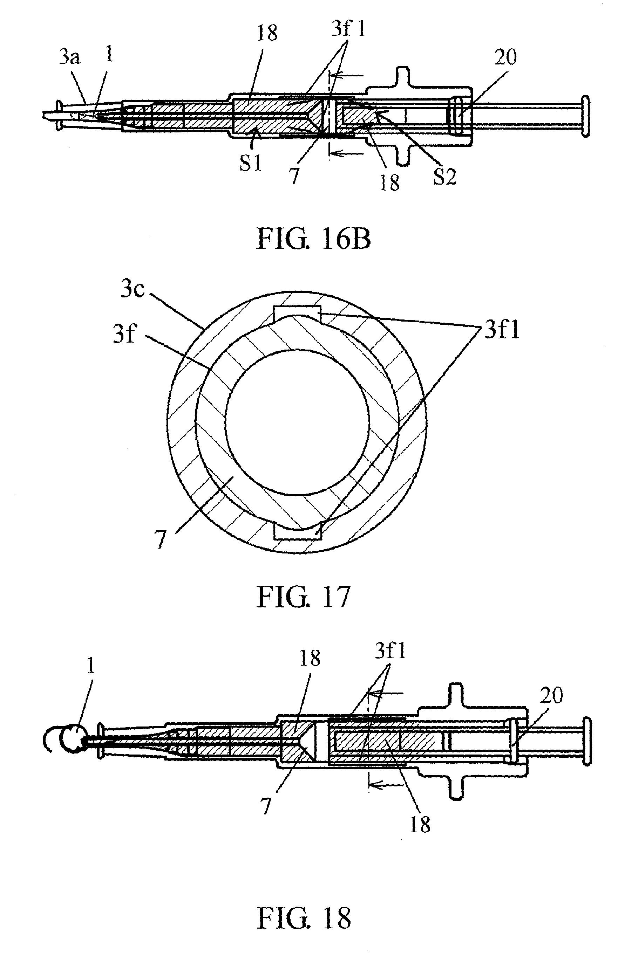

As shown in FIG. 16B, when the lens 1 is moved toward the front end opening 3j in the nozzle portion 3a by further pushing the pushing shaft 4 (during the movement of the lens 1 in the nozzle portion 3a), the seal cap 7 is located at an intermediate position of the groove portions 3f1 in the axial direction. Thereby, the groove portions 3f1 face the first area S1 in the main body 3 and a second area S2 closer to the rear end than the seal cap 7 in the main body 3. Therefore, the liquid 18 flows in the main body 3 from the first area S1 to the second area S2 through the groove portions 3f1. The liquid 18 flowing into the second area S2 does not flow out to the outside of the main body 3 due to the rear end opening 3e of the main body 3 being closed as described above.

FIG. 17 shows a sectional view orthogonal to the axial direction in the state of FIG. 16B in which the liquid flow path is opened (position of the section is shown by a dashed line in FIG. 16B). It is necessary to set a width and a depth of the groove portions 3f1 such that the groove portions 3f1 are not closed due to an elastic deformation of the seal cap 7.

In this way, the groove portions 3f1 serve as a liquid flow path through which the liquid 18 is caused to flow inside the main body 3 from the first area S1 to the second area S2. When the lens 1 is moved toward the front end opening 3j in the nozzle portion 3a, the liquid flow path (3f1) is opened. This structure makes it possible to perform smooth pushing of the pushing shaft 4 and pushing-out of the lens 1. The term "liquid flow path" does not include the front end opening 3j.

Further, as described above, the liquid 18 flowing from the first area in the main body S1 into the second area in the main body S2 does not flow out to the outside of the main body 3, but stays inside the main body 3. This makes it possible for the outer surface and a periphery of the insertion device 2 to not get wet by the liquid 18.

As shown in FIG. 18, when the lens 1 is ejected from the front end opening 3j of the nozzle portion 3a, the seal cap 7 is located closer to the front end than the groove portions 3f1. Thereby, in the main body 3, the liquid flow path from the first area S1 to the second area S2 formed by the groove portions 3f1 is shut off. That is, the first area S1 is brought into a state of being opened only through the front end opening 3j. In this state, since the inside of the first area S1 is filled with the liquid 18, even in a case where the pressure in the eyeball is higher than the pressure in the first area S1, the aqueous liquid in the eyeball does not flow back to the inside of the main body 3. Thereby, the pressure in the eyeball is maintained, and therefore the space in the anterior chamber is not greatly reduced, which hardly causes the movement in the iris.

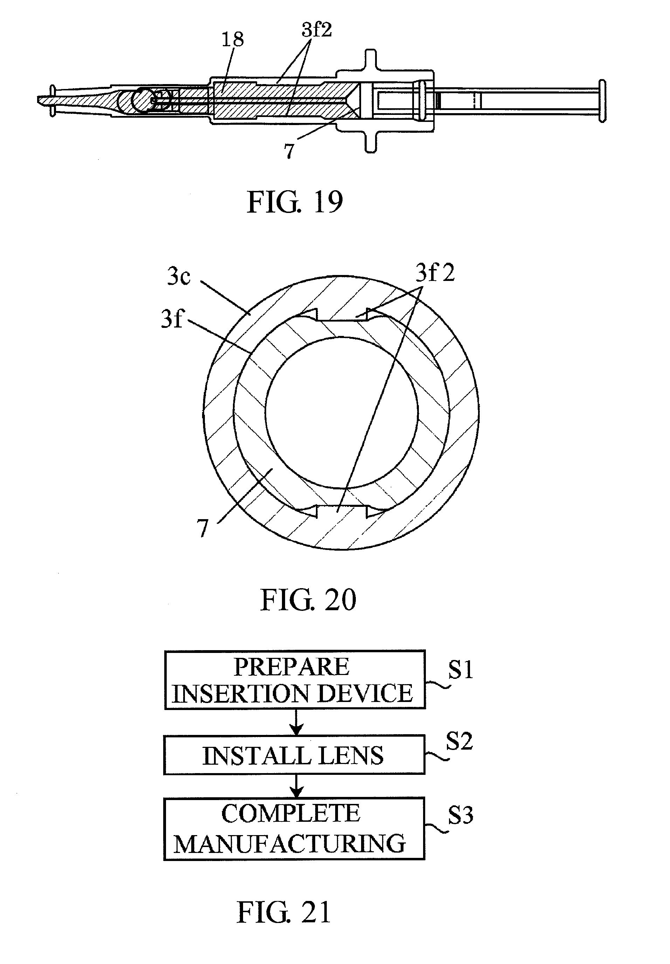

The case has been described where the liquid flow path is formed by forming the groove portions 3f1 in the inner wall of the main body 3. However, as shown in FIGS. 19 and 20, a liquid flow path may be formed by forming protrusions 3f2 extending in the axial direction on the inner wall (first inner circumferential surface 3f) of the main body 3, and the liquid flow path thus formed may be opened by the protrusions 3f2.

That is, the seal cap 7 is deformed in the radial directions by the protrusions 3f2, which forms gaps between the first inner circumferential surface 3f and the seal cap 7. Use of these gaps as liquid flow paths provides the same advantageous effect as that in the case where the groove portions 3f1 are formed.

In order to avoid the resistance against the pushing of the pushing shaft 4 from extremely increasing when the seal cap 7 rides over the protrusions 3f2, a slop shape is provided to a rear end side portion of each of the protrusions 3f2 to suppress the increase of the resistance.

This embodiment has described the case where the groove portions 3f1 or the protrusions 3f2 are formed in two places facing each other in the first inner circumferential surface 3f. However, the number and positions of the groove portions 3f1 and the protrusions 3f2 can be arbitrarily selected, and those may be formed in one or more places.

FIG. 21 shows a method for manufacturing the intraocular lens preloaded insertion device 2 of each of Embodiments 1 and 2. The insertion device 2 is manufactured through a step (step S1) of preparing the insertion device (the main body 3, the pushing shaft 4, the lens holding member 5 and others) before housing the lens 1 therein, and a step (step S2) of housing and holding, i.e., of installing the lens 1 in the lens housing portion 3b. The insertion device 2 in which the lens 1 is housed is sterilized and packaged, which completes the manufacturing process (step S3).

As described above, according to the above-described respective embodiments, even in the state where most of or entire inner space of the nozzle portion 3a is occupied by the lens 1 moving in the nozzle portion 3a, it is possible to cause the liquid in the main body 3 to flow out to the outside or to flow to another area (from the first area S1 to the second area S2) in the main body 3 because the liquid flow path not including the front end opening 3j of the nozzle portion 3a is opened,. Therefore, it is possible to perform the pushing of the pushing shaft 4, i.e., the pushing-out of the lens 1 into the eye smoothly. Additionally, since the liquid flow path is shut off when the lens 1 is ejected through the front end opening 3j of the nozzle portion 3a, even if the pressure in the main body 3 is decreased to be lower than the pressure in the eye, it is possible to prevent the aqueous liquid in the eyeball from flowing back to the inside of the main body 3.

While the present invention has been described with reference to exemplary embodiments, it is to be understood that the invention is not limited to the disclosed exemplary embodiments. The scope of the following claims is to be accorded the broadest interpretation so as to encompass all such modifications, equivalent structures and functions.

The present invention can provide an insertion device for intraocular lens capable of smoothly performing pushing-out of the lens into an eye by a pushing shaft in a state in which a main body is filled with liquid, and of preventing aqueous fluid in the eyeball from flowing back to an inside of the insertion device.

## Claims

1. An insertion device for inserting an intraocular lens into an eye, comprising:
a main body configured to include a lens housing portion in which the lens is housed and an insertion cylindrical portion for ejecting the lens into the eye through its front end opening;
a pushing shaft configured to move the lens from the lens housing portion to push out the lens into the eye through the insertion cylindrical portion; and
a liquid flow path other than the front end opening that is to cause liquid to flow from the inside of the main body to the outside or inside the main body,
wherein the insertion device is configured to open the liquid flow path when the lens moves toward the front end opening in the insertion cylindrical portion, and to shut off the liquid flow path after the lens is ejected from the front end opening and inserted into the eye.

2. An intraocular lens preloaded insertion device comprising:
an insertion device according to claim 1; and
an intraocular lens held in the lens housing portion of the insertion device.

3. A method for manufacturing an intraocular lens preloaded insertion device comprising the steps of:
preparing an insertion device according to claim 2; and
causing the lens housing portion of the insertion device to hold the intraocular lens.
